# EUROPEAN PATENT APPLICATION

(11) **EP 3 569 239 A2**
(43) Date of publication of application: **20.11.2019**
(21) Application number: 18739347.5
(22) Date of filing: 11.01.2018
(51) Int. Cl.: A61K 35/54, A61K 35/28, A61K 8/98, A61Q 7/00

(54) **METHOD FOR PREPARING COMPOSITION, INCLUDED WITHIN EXOSOME OBTAINED FROM NANOG-INTRODUCED, FETUS-DERIVED MESENCHYMAL STEM CELL IN AMNIOTIC FLUID, FOR HAIR GROWTH**

(30) Priority: 11.01.2017 KR 20170004242
(71) Applicant: Stemlab Inc., Seongbuk-gu Seoul 02841 (KR)
(72) Inventor: YOU, Seung Kwon, Yongin-si Gyeonggi-do 17163 (KR); PARK, Jung-Hyun, Seoul 05315 (KR); JANG, Ji-Hoon, Jeonju-si Jeollabuk-do 55086 (KR); JUN, Eun Kyoung, Seoul 02798 (KR)
(74) Representative: Eisenführ Speiser
(86) International application number: PCT/KR2018/000506
(87) International publication number: WO 2018/131900

(57) **Abstract**

The present invention relates to a composition for hair growth and a preparation method therefor through the separation of exosomes from a culture medium of fetus-derived mesenchymal stem cells overexpressing the dedifferentiation factor Nanog in amniotic fluid.

<110>     StemLab, Inc.

<120>     METHOD FOR PRODUCING EXOSOME COMPOSITION FOR PROMOTING HAIR
          GROWTH FROM HUMAN AMNIOTIC FLUID-DERIVED MESENCHYMAL STEM CELLS
          WITH NANOG

<130>     X17E10D0246

<150>     KR 10-2017-0004242
<151>     2017-01-11

<160>     17

<170>     KoPatentIn 3.0

<210>     1
<211>     918
<212>     DNA
<213>     Homo sapiens

<400>     1
atgagtgtgg atccagcttg tccccaaagc ttgccttgct ttgaagcatc cgactgtaaa          60

gaatcttcac ctatgcctgt gatttgtggg cctgaagaaa actatccatc cttgcaaatg         120

tcttctgctg agatgcctca cacggagact gtctctcctc ttccttcctc catggatctg         180

cttattcagg acagccctga ttcttccacc agtcccaaag gcaaacaacc cacttctgca         240

gagaagagtg tcgcaaaaaa ggaagacaag gtcccggtca agaaacagaa gaccagaact         300

gtgttctctt ccacccagct gtgtgtactc aatgatagat ttcagagaca gaaatacctc         360

agcctccagc agatgcaaga actctccaac atcctgaacc tcagctacaa acaggtgaag         420

acctggttcc agaaccagag aatgaaatct aagaggtggc agaaaaacaa ctggccgaag         480

aatagcaatg gtgtgacgca gaaggcctca gcacctacct accccagcct ttactcttcc         540

taccaccagg gatgcctggt gaacccgact gggaaccttc caatgtggag caaccagacc         600

tggaacaatt caacctggag caaccagacc cagaacatcc agtcctggag caaccactcc         660

tggaacactc agacctggtg cacccaatcc tggaacaatc aggcctggaa cagtcccttc         720

tataactgtg gagaggaatc tctgcagtcc tgcatgcagt tccagccaaa ttctcctgcc         780

agtgacttgg aggctgcctt ggaagctgct ggggaaggcc ttaatgtaat acagcagacc         840

actaggtatt ttagtactcc acaaaccatg gatttattcc taaactactc catgaacatg         900

caacctgaag acgtgtga                                                       918


<210>     2
<211>     17
<212>     DNA
<213>     Artificial Sequence

<220>
<223>     Exo-Nanog foward

<400>    2
gcttggatac acgccgc                                                      17


<210>    3
<211>    20
<212>    DNA
<213>    Artificial Sequence

<220>
<223>    Exo-Nanog reverse


<400>    3
gattgttcca ggattgggtg                                                   20


<210>    4
<211>    20
<212>    DNA
<213>    Artificial Sequence

<220>
<223>    bFGF forward


<400>    4
cagattagcg gacgcggtgc                                                   20


<210>    5
<211>    20
<212>    DNA
<213>    Artificial Sequence

<220>
<223>    bFGF reverse


<400>    5
tcacggatgg gtgtctccgc                                                   20


<210>    6
<211>    24
<212>    DNA
<213>    Artificial Sequence

<220>
<223>    IGF forward


<400>    6
ccatgtcctc ctcgcatctc ttct                                              24


<210>    7
<211>    23
<212>    DNA
<213>    Artificial Sequence

<220>
<223>    IGF reverse

<400>    7
ccataccctg tgggcttgtt gaa                                                23

<210>    8
<211>    22
<212>    DNA
<213>    Artificial Sequence

<220>
<223>    Wnt7a forward

<400>    8
tctttctcag cctgggcatg gt                                                 22

<210>    9
<211>    22
<212>    DNA
<213>    Artificial Sequence

<220>
<223>    Wnt7a reverse

<400>    9
tcctatgacg atgatggcgt cg                                                 22

<210>    10
<211>    22
<212>    DNA
<213>    Artificial Sequence

<220>
<223>    PDGF-AA forward

<400>    10
ctgcccattc ggaggaagag aa                                                 22

<210>    11
<211>    23
<212>    DNA
<213>    Artificial Sequence

<220>
<223>    PDGF-AA reverse

<400>    11
tggcacttga cactgctcgt gtt                                                23

<210>    12
<211>    22
<212>    DNA
<213>    Artificial Sequence

<220>
<223>    ALP forward


<400>    12
tggccctctc caagacgtac aa                                                22


<210>    13
<211>    22
<212>    DNA
<213>    Artificial Sequence

<220>
<223>    ALP reverse


<400>    13
tggttcactc tcgtggtggt ca                                                22


<210>    14
<211>    21
<212>    DNA
<213>    Artificial Sequence

<220>
<223>    LEF forward


<400>    14
cttccttggt gaacgagtct g                                                 21


<210>    15
<211>    20
<212>    DNA
<213>    Artificial Sequence

<220>
<223>    LEF reverse


<400>    15
gtgttctctg gccttgtcgt                                                   20


<210>    16
<211>    22
<212>    DNA
<213>    Artificial Sequence

<220>
<223>    Versican forward


<400>    16
aactagccgt tggagtggat tc                                                22


<210>    17
<211>    20
<212>    DNA

<213>    Artificial Sequence

<220>
<223>    Versican reverse

<400>    17
aaatgctctg tggctctgga                                                    20

## Description

### [Technical Field]

The present invention relates to a composition for promoting hair growth through isolation of exosomes in a culture solution of mesenchymal stem cells derived from a fetus in amniotic fluid in which Nanog, which is a reprogramming factor, is overexpressed, and a method of preparing the composition.

### [Background Art]

Stem cells refer to cells that have an infinite self-renewal ability under *in vivo* characteristic conditions and environments or according to the degree of need in the cells themselves and have an ability to differentiate into specific cells and tissues needed in the body. There are three types of stem cells: embryonic stem cells (ES cells) isolated from early embryos, embryonic germ cells (EG cells) isolated from primordial germs cells in the embryonic phase, and multipotent adult progenitor cells (MAPC cells) isolated from adult bone marrow.

Among these stem cells, mesenchymal stem cells, which are well known as adult stem cells, have already been variously utilized as a cell therapeutic agent and a culture solution therapeutic agent clinically, and effects thereof have also been proven to a great extent. It has been confirmed that such mesenchymal stem cells can be isolated from various body tissues, and these cells can be identified in, particularly, bone marrow, fat, umbilical cord blood, amniotic fluid, and the like. It is also well known that the nature and characteristics of these cells are identical to a great extent and availability thereof as therapeutic agents is also similar or identical.

Among these cells, amniotic fluid-derived mesenchymal stem cells have various advantages, and thus have excellent value in terms of research and commercial availability. Amniotic fluid-derived mesenchymal stem cells can be isolated from maternal amniotic fluid and cultured *in vitro,* and amniotic fluid may be obtained easily from a pregnant woman. In addition, amniotic fluid is used for examining various pieces of information about fetal health. It can be used for research purposes by consent of a patient and a method of acquiring amniotic fluid is also very simple such that it can be cultured *in vitro* on the day of acquisition. Due to these advantages, the use of amniotic fluid-derived mesenchymal stem cells as a therapeutic agent may be determined as reasonable.

In human hair, a certain number of hairs are maintained in a required site. Hair has a unique growth cycle, which is referred to as a hair cycle including: anagen phase -> catagen phase -> telogen phase -> exogen phase. This mechanism is controlled such that dermal papilla cells, which are located near the hair root, deliver a signal to the surrounding cells, and a growth factor received by the cells and the number of the cells themselves play a major role in hair growth. When hair loss progresses, dermal papillae become smaller, which causes a decrease in the thickness of hair, and the hair cycle is also shortened and newly grown hair becomes thinner. As this process progresses, baldness progresses, and hair turns into fluffy hair and the hair growth cycle becomes shorter, and thus the hair falls out after slightly growing. The major cause of baldness is heredity, and it has also been reported that a male hormone, testosterone, inhibits the growth of dermal papilla cells to thereby induce hair loss. In addition to these *in vivo* causes, hair loss occurs by various environmental factors. In particular, it is known that aging-related hair loss is caused by poor supply of oxygen to follicular tissues due to a decrease in scalp cells and an increase in the accumulation amount of scalp fat and a consequent problem in providing nutrients for follicular tissues. In addition, there are also many opinions that external factors such as stress, irregular lifestyles, environmental pollution, and the like may cause hair loss.

While individuals with baldness or hair loss feel great emotional pain, most hair restorers, which have been developed and are currently commercially available, have temporary or limited effects, and thus do not sufficiently satisfy users' needs. An applicable hair restorer, minoxidil, which has been proven to be effective to some extent and currently used, and an oral hair restorer, Propecia, which contains finasteride as an active ingredient, are widely used as agents having an excellent hair loss-preventing effect, respectively due to vasodilation and due to an inhibitory action on activation of a male hormone. However, the above-described hair restorers are effective in preventing hair loss to some extent, but exhibit insignificant effects on hair growth. That is, when the use of these hair restorers is stopped, hair loss reoccurs, and there are great concerns about side effects and increased costs when used long-term, and thus most patients are giving up treatment.

Therefore, the inventors of the present invention isolated and acquired, up to an exosome unit, a conditioned medium isolated from mesenchymal stem cells derived from a fetus in amniotic fluid which promoted cell growth and prolonged a lifespan of cells through introduction of Nanog thereinto, evaluated the effect thereof on hair growth, and selected major factors therefor.

### [Cited References]

### [Patent Documents]

Korean Patent Publication No. 2013-0116972
Korean Patent Registration No. 1422559

### [Disclosure]

### [Technical Problem]

An object of the present invention is to provide a method of producing exosomes including a human growth factor produced from Nanog-overexpressing mesenchymal stem cells derived from a fetus in amniotic fluid.

Another object of the present invention is to provide a composition for promoting hair growth, which includes, as an active ingredient, exosomes including a human growth factor produced from Nanog-overexpressing mesenchymal stem cells derived from a fetus in amniotic fluid, and a cosmetic or pharmaceutical composition therefor.

### [Technical Solution]

According to an aspect of the present invention, there is provided a method of producing exosomes derived from mesenchymal stem cells, including:
(a) isolating fetus-derived cells from amniotic fluid obtained from a pregnant woman;
(b) sub-culturing the isolated fetus-derived cells in a medium containing fetal bovine serum (FBS) and basic fibroblast growth factor (bFGF) to obtain mesenchymal stem cells derived from a fetus in amniotic fluid;
(c) introducing Nanog into the mesenchymal stem cells derived from a fetus in amniotic fluid to obtain Nanog-overexpressing mesenchymal stem cells derived from a fetus in amniotic fluid;
(d) culturing the obtained Nanog-overexpressing mesenchymal stem cells derived from a fetus in amniotic fluid for 1 day to 5 days to prepare a conditioned medium; and
(e) isolating exosomes from the conditioned medium.

The term "mesenchymal stem cells (MSCs)" as used herein refers to cells from which bone, cartilage, fat, bone marrow stroma, muscle, nerve, or the like originates, and cells that generally remain in the bone marrow in adults, but also are present in cord blood, peripheral blood, other tissues, and the like, and obtainable therefrom. Since amniotic fluid, which is obtained from a pregnant woman, includes various chemicals generated from a fetal body, most cells in the human body may be generated, and sampling thereof is relatively easy. In addition, it was confirmed that heterogenous cells are present in amniotic fluid, and among these cells, there are homogenous MSCs having the same shape as that of fibroblasts, which is a feature of MSCs.

The term "Nanog" as used herein, which is a reprogramming factor, originates from "reprogramming," which is a concept introduced by Professor Yamanaka and his team in 2006. All adult tissues gradually become differentiated in an undifferentiated state through a normal development process, and are changed to functionally-specified cells. Among these, cells of the fertilized egg are totipotent, and as the subsequent development processes progress, the cells become blastocysts, which can be divided into inner cell masses and outer cells. In this case, the inner cell masses may generate embryonic somatic cells and germ cells, which is called pluripotency. The embryonic stem cells exhibit a pluripotency-specific gene expression pattern, and examples thereof include Oct4, Sox2, Nanog, Lin28, and the like. Reprogramming may be a technology of inducing such specific gene expression in somatic cells to thereby return the cells to have properties similar to those of embryonic stem cells. Nanog is one of various factors that have been used in a series of studies, and the researchers of the present invention utilized Nanog as a technology that induces overexpression by introducing a gene into cells via a viral vector.

The term "culture medium" as used herein refers to a medium that can sustain the growth and survival of cells derived from a fetus in amniotic fluid *in vitro,* and includes all media commonly used in the art, which are suitable for culturing cells derived from a fetus in amniotic fluid. Medium and culture conditions may be selected depending on the type of cells. The medium used in culture is preferably a cell culture minimum medium (CCMM), and generally includes a carbon source, a nitrogen source, and trace elements. Examples of the CCMM include, but are not limited to, Dulbecco's Modified Eagle's Medium (DMEM), Minimal Essential Medium (MEM), Basal Medium Eagle (BME), RPMI1640, F-10, F-12, α Minimal Essential Medium (αMEM), Glasgow's Minimal Essential Medium (GMEM), and Iscove's Modified Dulbecco's Medium (IMEM). In addition, the CCMM may include an antibiotic such as penicillin, streptomycin, gentamicin, or the like.

In the present invention, the MSCs derived from a fetus in amniotic fluid may be obtained by culturing the cells isolated from the amniotic fluid in a basal medium containing FBS and bFGF, and preferably, may be obtained by culturing the cells in 10% FBS-containing low-glucose DMEM supplemented with 4 ng/ml of bFGF, 5 ng/ml of selenium, and 50 µg/ml of ascorbic acid. In an exemplary embodiment of the present invention, the low-glucose DMEM may further include 10% FBS, 1% L-glutamine and 1% penicillin-streptomycin, and a solution of 4 ng/ml of bFGF, 5 ng/ml of seleniuim, and 50 µg/ml of ascorbic acid.

In process (c), the introduction of the Nanog may be performed using a retroviral vector.

More specifically, in process (d), the conditioned medium may be prepared by culturing the MSCs obtained in process (c) in a serum-free medium for 3 days.

In particular, the present invention provides a method of performing manipulation by culturing Nanog-introduced fetus-derived MSCs in a suitable medium to synthesize Apo-1/Fas, epidermal growth factor (EGF), Interferon-γ inducible protein-10 (IP-10), leptin, MIP4, MMP3, Rantes, interferon-γ (IFNγ), human transforming growth factor-β (TGF-β), tumor necrosis factor-α (TNFα), tumor necrosis factor receptor I (TNFR I), tumor necrosis factor receptor II (TNFRII), intercellular adhesion molecule 1 (ICAM-1), vascular cell adhesion molecule-1 (VCAM-1), vascular endothelial growth factor (VEGF), interleukin-1β (IL-1β), interleukin-1 receptor α (IL-1Rα), IL-2, IL-3, IL-4, IL-5, IL-6, IL-6R, IL-7, IL-8, IL-12, IL-15, and the like, more preferably, a hair growth-promoting growth factor such as basic fibroblast growth factor (bFGF), insulin-like growth factor (IGF), platelet-derived growth factor (PDGF)-AA, Wnt7a, or the like. Ingredients may be identified using the conditioned medium of the MSCs derived from a fetus in amniotic fluid through enzyme-linked immunosorbent assay (ELISA) and may be confirmed *in vitro.*

In the present invention, to obtain the conditioned medium of fetus-derived MSCs, MSCs isolated from amniotic fluid may be cultured in a serum-free medium containing a DMEM nutrient mixed solution including amino acids or analogs thereof and vitamins and analogs thereof. In this regard, it is possible to add an oxidative nutrient such as L-glutamine or the like, an energy metabolite such as sodium pyruvate or the like, and a carbon control source such as sodium bicarbonate or the like. The mixed solution is prepared by mixing various minerals and amino acids that assist in maintaining the growth and homeostasis of cells and promote cell stability and retention in sub-culturing after initial culturing of MSCs, vitamin-based nutrients capable of promoting the production of growth factors secreted from fetus-derived MSCs, and other factors, at a certain ratio.

The term "conditioned medium" as used herein refers to a medium prepared by subjecting cells to liquid suspension culture, removing divided cells when the cells reach the exponential phase, which is the cell division golden age, by centrifugation or filtration, collecting only a culture solution, and mixing the culture solution with a culture substrate. This medium uses an unknown growth factor extracted from dividing cells in the culture medium, and is widely used in low-density cell plating or protoplast culture.

The conditioned medium of the present invention abundantly contains substances such as growth factors derived from fetus-derived MSCs, and preferably includes Apo-1/Fas, epidermal growth factor (EGF), Interferon-y inducible protein-10 (IP-10), leptin, MIP4, MMP3, Rantes, interferon-γ (IFNγ), human transforming growth factor-β (TGF-β), tumor necrosis factor-α (TNFα), tumor necrosis factor receptor I (TNFR I), tumor necrosis factor receptor II (TNFR II), intercellular adhesion molecule 1 (ICAM-1), vascular cell adhesion molecule-1 (VCAM-1), vascular endothelial growth factor (VEGF), interleukin-1β (IL-1β), interleukin-1 receptor α (IL-1Rα), IL-2, IL-3, IL-4, IL-5, IL-6, IL-6R, IL-7, IL-8, IL-12, and IL-15. Most preferably, the conditioned medium includes basic fibroblast growth factor (bFGF), insulin-like growth factor (IGF), platelet-derived growth factor (PDGF)-AA, Wnt7a, and the like, which are hair growth-promoting growth factors.

A major limitation in the production of conditioned media of MSCs is largely related to the aging of stem cells. Aging of cells induces cell growth arrest and reduces the amounts of secreted growth factors and cytokines and causes cell death. In addition, it causes quantitative and qualitative losses of a cell-derived conditioned medium and makes it difficult to maintain a uniform composition, which results in difficulty in mass-production and commercialization of the conditioned medium and a composition including the conditioned medium as an active ingredient. Therefore, the inventors of the present invention have introduced the reprogramming technology to MSC culture in previous studies. According to the preceding studies, various pluripotent factors such as Oct4, Nanog, and Lin28 were introduced into amniotic fluid-derived MSCs, and it was confirmed that, among these factors, Nanog-introduced amniotic fluid-derived MSCs could be established as a cell line and continuously cultured, whereas the introduction of the other genes caused cell death and continuous cell culture was difficult. Furthermore, it was confirmed that the effect of Nanog-introduced stem cells on promoting cell growth and preserving sternness was greater than that of existing amniotic stem cells. From the above results, it was verified that it was possible to continuously prepare a conditioned medium of Nanog-introduced amniotic fluid-derived stem cells and obtain large amounts of growth factors included in the medium.

The term "exosomes" as used herein refers to nano-sized circular vesicles consisting of a phospholipid membrane, as a delivery system material for intercellular interactions. Exosomes generally include mRNA, shRNA, proteins, and the like internally, and these substances are transferred inside and outside cells to perform their functions. Externally secreted exosomes deliver the substances to other cells, and cells having received the substances exhibit a reaction mechanism corresponding thereto. To date, various functions of exosomes have been researched, and cancer biomarkers, wound healing, protein transfer, shRNA delivery, and the like are generally known. When compared to cell secretomes, exosomes are highly resistant to the extracellular environment and also have excellent adsorption to other cells. In addition, exosomes are sufficiently valuable in terms of availability as a functional agent since the concentration thereof is easily controlled.

Specifically, the exosomes isolated in process (e) may include any one or more human growth factors selected from the group consisting of basic fibroblast growth factor (bFGF), insulin-like growth factor (IGF), wingless-type MMTV integration site family member 7A (Wnt7a), and platelet-derived growth factor (PDGF)-AA, but the present invention is not limited thereto.

In one embodiment of the present invention, it was confirmed that the isolated exosomes contain mRNA that is related to hair growth factors and a great number of growth factors and the like, and, when treating dermal papilla cells with the exosomes, the exosomes not only promoted the growth of the dermal papilla cells, but also activated the hair growth signaling pathway.

In addition, in one embodiment of the present invention, it was confirmed that, as a result of removing exosomes from a conditioned medium and then analyzing growth factors and the like, growth factors and the like were significantly reduced after the removal of exosomes, and large amounts of growth factors and the like were contained in the exosomes. Accordingly, when isolated exosomes were used, the possibility of more efficiently utilizing growth factors and the like included in the exosomes was confirmed.

The present invention also provides a cosmetic composition for promoting hair growth, which includes, as an active ingredient, exosomes isolated from MSCs derived from a fetus in amniotic fluid.

The cosmetic composition may be variously used in hair cosmetic products that are effective in promotion of hair growth, hair regeneration, and hair loss prevention.

The amount of the exosomes isolated from MSCs may range from 0.001 part by weight to 10 parts by weight with respect to 100 parts by weight of the cosmetic composition.

The cosmetic composition may include a lipid material, an organic solvent, a solubilizer, a thickener, a gelling agent, a softener, an antioxidant, a suspending agent, a stabilizer, a foaming agent, a fragrance, a surfactant, water, an ionic or non-ionic emulsifier, a filler, a metal ion sequestering agent, a chelating agent, a preservative, a vitamin, a blocking agent, a wetting agent, an essential oil, a dye, a pigment, a hydrophilic or lyophilic activating agent, lipid vesicles, or an additive commonly used in the cosmetic or dermatological field, such as any other components commonly used in cosmetics. The additive is introduced in an amount generally used in the cosmetic or dermatological field.

An external form of the cosmetic composition includes a cosmetically or dermatologically acceptable medium or base. The medium or base may be formulated in all preparations suitable for topical application, for example, a solution, a gel, a solid, a paste anhydrous product, an emulsion obtained by dispersing an oil phase in water phase, a suspension, a microemulsion, a microcapsule, microgranules, an ionic (liposome) and non-ionic vesicle dispersant, a cream, a skin softener, a lotion, a powder, an ointment, a spray, or a concealer stick. The composition may be prepared using a method commonly used in the art. The composition according to the present invention may also be used in the form of a foam or an aerosol composition further including a compressed propellant.

The cosmetic composition of the present invention is not particularly limited in terms of its formulation, and may be formulated as a cosmetic product, for example, a moisturizing lotion, an astringent, a nourishing lotion, a nourishing cream, a massage cream, an essence, an eye cream, an eye essence, a cleansing cream, a cleansing foam, a cleansing water, a pack, a powder, a body lotion, a body cream, a body oil, a body essence, a hair tonic, a hair conditioner, a hair essence, a hair lotion, a hair nourishing lotion, a hair shampoo, a hair rinse, a hair treatment, a hair cream, a hair nourishing cream, a hair moisturizing cream, a hair massage cream, a hair wax, a hair aerosol, a hair pack, a hair nourishing cream, a hair moisturizing cream, a hair massage cream, a hair wax, a hair aerosol, a hair pack, a hair nourishing pack, a hair soap, a hair cleansing foam, a hair oil, a hair drying preparation, a hair preservation treatment, a hair colorant, a hair waving preparation, a hair decolorant, a hair gel, a hair glaze, a hair dressing, a hair lacquer, a hair moisturizer, a hair mousse, or a hair spray.

The cosmetic composition as described above may be applied onto the skin, or applied to be absorbed into the skin using microneedles or the like.

The present invention also provides a pharmaceutical composition for promoting hair growth, which includes, as an active ingredient, exosomes isolated from MSCs derived from a fetus in amniotic fluid.

The pharmaceutical composition may be variously used in hair medical products that are effective in promotion of hair growth, hair regeneration, and hair loss prevention.

The amount of the exosomes isolated from MSCs may range from 0.001 part by weight to 10 parts by weight with respect to 100 parts by weight of the pharmaceutical composition.

The pharmaceutical composition may be prepared in the form of tablets, capsules, powders, granules, injections, gels, emulsions, syrups, aerosols, patches, sprays, creams, ointments, plasters, lotions, liniments, pastes, or cataplasmas for promoting hair growth, but the present invention is not limited thereto.

When the pharmaceutical composition is formulated, a diluent or an excipient, such as a filler, a thickener, a binder, a wetting agent, a disintegrating agent, a surfactant, or the like is generally used. Solid preparations for oral administration include tablets, pills, powders, granules, capsules, and the like, and are prepared by mixing one or more compounds with at least one excipient, for example, starch, calcium carbonate, sucrose, lactose, gelatin, or the like. In addition, other than simple excipients, lubricants such as magnesium stearate, talc, and the like are also used. Liquid preparations for oral administration may include suspending agents, liquids for internal use, emulsions, syrups, and the like, and other than frequently used simple diluents such as water or liquid paraffin, various excipients, e.g., wetting agents, sweetening agents, fragrances, preservatives, and the like. Preparations for parenteral administration include sterilized aqueous solutions, non-aqueous solvents, suspending agents, emulsions, lyophilizing agents, and suppositories. As a non-aqueous solvent or a suspending solvent, propylene glycol, polyethylene glycol, a vegetable oil such as olive oil, or an injectable ester such as ethyl oleate may be used. As the base of a suppository, Witepsol, Macrogol, Tween 61, cacao butter, laurin, or glycerogelatin may be used.

The pharmaceutical composition may be administered as a pharmaceutically acceptable salt thereof, alone or in combination with a different pharmaceutically active compound, or a suitable combination thereof. The salt is not particularly limited and may be any pharmaceutically acceptable salt, and for example, hydrochloric acid, sulfuric acid, nitric acid, phosphoric acid, hydrofluoric acid, hydrobromic acid, formic acid, acetic acid, tartaric acid, lactic acid, citric acid, fumaric acid, maleic acid, succinic acid, methanesulfonic acid, benzenesulfonic acid, toluenesulfonic acid, or naphthalene sulfonic acid may be used.

The pharmaceutical composition may be administered parenterally or orally according to the purpose of use and administered once or several times at a daily dose of 0.1 mg/kg (body weight) to 500 mg/kg (body weight), for example, 1 mg/kg (body weight) to 100 mg/kg (body weight). A dosage for a specific patient may vary depending on the body weight, age, and gender of a patient, health condition, diet, administration time, administration method, excretion rate, the severity of a disease, and the like.

The pharmaceutical composition may be administered via various routes, such as parenterally or orally, to mammals such as rats, mice, livestock, humans, and the like, and all administration methods may be predicted, for example, oral injection, rectal or intravenous injection, intramuscular injection, subcutaneous injection, intrauterine injection, or intracerebroventricular injection.

### [Advantageous Effects]

According to the present invention, by introducing Nanog as a reprogramming factor into MSCs derived from a fetus in amniotic fluid, the growth, sternness, and lifespan of the MSCs were enhanced, and the expression of growth factors secreted therefrom was enhanced. In addition, exosomes isolated from a conditioned medium obtained by culturing Nanog-introduced MSCs derived from a fetus in amniotic fluid also exhibit a hair growth promotion effect, and thus can be used as a cosmetic or pharmaceutical composition for promoting hair growth.

### [Description of Drawings]

FIG. 1 illustrates results of confirming whether Nanog was overexpressed in cell lines according to an embodiment of the present invention.
FIG. 2 illustrates results of measuring average sizes and numbers of amniotic fluid-derived MSCs and Nanog-introduced amniotic fluid-derived MSCs, according to an embodiment of the present invention.
FIG. 3 illustrates analysis results of substances inside exosomes through PCR, according to an embodiment of the present invention.
FIG. 4 illustrates analysis results of substances inside exosomes through ELISA, according to an embodiment of the present invention.
FIG. 5 illustrates results of confirming functions of exosomes obtained according to an embodiment of the present invention.
FIG. 6 illustrates AP staining results of confirming changes in hair growth markers along with the growth of hair papillae by exosomes obtained according to an embodiment of the present invention.
FIG. 7 illustrates results of confirming changes in markers of exosomes obtained according to an embodiment of the present invention.

### [Best Mode]

Unless otherwise defined, technical and scientific terms as used herein have the same meaning as commonly understood by one of ordinary skill in the art to which this invention pertains. Generally, the nomenclature used herein is well known in the art and commonly used.

### [Examples]

### Example 1: Establishment of Nanog-Introduced MSCs Derived from Amniotic Fluid

To obtain amniotic fluid-derived MSC lines, which are easy for cell expansion, the Nanog gene was introduced into amniotic fluid-derived MSCs using a retroviral vector system to induce Nanog overexpression, thereby establishing Nanog-introduced amniotic fluid-derived MSC lines. A detailed method is as follows.

Fetus-derived cells isolated from amniotic fluid obtained from pregnant women were sub-cultured in a low glucose Dulbecco's Modified Eagle's Medium (DMEM) supplemented with 10% fetal bovine serum (FBS), 1% penicillin/streptomycin, 1% L-glutamine, 4 ng/ml of basic fibroblast growth factor (bFGF), 5 ng/ml of selenium, and 50 µg/ml of ascorbic acid to obtain MSCs derived from a fetus in the amniotic fluid.

The Nanog gene was introduced into the obtained amniotic fluid-derived MSCs using a retroviral vector system to induce Nanog overexpression, thereby completing the preparation of Nanog-introduced amniotic fluid-derived MSC lines.

Specifically, a restriction enzyme was used to isolate the Nanog gene (NCBI GenBank Accession number NM_024865.3 (SEQ ID NO: 1) from a pBS-Nanog vector (Yamanaka lab's plasmid stock # A4, JAPAN). The isolated Nanog gene was ligated to a pMXs vector (Cell Biolab, JAPAN) with T4 ligase to produce pMXs-Nanog. The pMXs-Nanog vector was transfected into 293GPG cells for 6 hours using a transfection reagent. A virus with the Nanog gene was produced for 72 hours, and the supernatant was separated, centrifuged at 2000 rpm for 10 minutes, and then filtered through a 0.45-µm filter. The virus was infected into cells by treatment with amniotic fluid-derived MSCs in an 80% confluence state for 6 hours.

### Example 2: Verification of Nanog Expression of Nanog-Introduced Amniotic Fluid-Derived MSCs

For RT-PCR, total RNA was isolated from the Nanog-introduced amniotic fluid-derived MSCs using a TRIzol reagent (Invitrogen, USA) in accordance with the manufacturer's instructions. 500 µg of the isolated total RNA was reverse-transcribed with cDNA using a cDNA preparation mix (Bioneer). Test tubes were allowed to stand at 45 °C for 60 minutes and then at 95 °C for 5 minutes, and then stored at -20 °C until use. cDNA was amplified using a Taq polymerase (Promega) and primers specific to Nanog mRNA (Exo-Nanog foward: 5'-GCTTGGATACACGCCGC-3' (SEQ ID NO: 2); and Exo-Nanog reverse: 5'-GATTGTTCCAGGATTGGGTG-3' (SEQ ID NO: 3)). In the RT-PCR, a cycle of 94 °C for 20 seconds, 60 °C for 30 seconds, and 72 °C for 2 minutes was repeated 35 times, and then final synthesis proceeded at 72 °C for 10 minutes. The PCR products were electrophoresed on 1% agarose gel and analyzed.

For Western blot experiments to confirm Nanog overexpression, the Nanog-introduced amniotic fluid-derived MSCs were cultured in a DMEM containing 10% FBS to 100% confluency and then obtained, the cells were disrupted, and then 30 µg of a protein-containing supernatant was separated on sodium dodecyl sulfate-polyacrylamide gel electrophoresis (SDS-PAGE) gel and transferred to a nitrocellulose membrane, followed by blocking with 4% skim milk. Subsequently, the membrane was treated with an antibody (AF1997, R&D) for detecting Nanog receptors, as a primary antibody, cultured at 4 °C overnight, and washed with TBST (Tris-buffered saline (TBS) supplemented with 0.1% Tween 20). The membrane was treated with a goat-derived HRP antibody as a secondary antibody (Santa Cruz Biotechnology, USA) along with TBST containing 1% bovine serum albumin (BSA), and a reaction was allowed to occur therebetween for 1 hour, followed by western blotting. As a control for comparison in expression levels, the expression of α-tubulin was confirmed using the same method as described above by using an anti-α-tubulin antibody (R&D, USA) as a primary antibody.

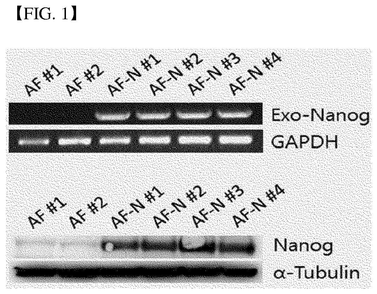

As a result, it was confirmed that the Nanog-introduced amniotic fluid-derived MSCs exhibited significant increases in an mRNA expression level of the exogenous Nanog gene and a protein expression level of Nanog, as compared to amniotic fluid-derived MSCs (see FIG. 1).

When the fetus-derived MSCs were cultured in DMEM low Glucose + 10% fatal bovine serum +1% penicillin/streptomycin +1% non-essential amino acids + 4 ng/ml basic FGF (bFGF) + 5 ng/ml selenium + 50 µg/ml ascorbic acid), continuous cell growth occurred.

### Example 3: Analysis of Exosomes in Conditioned Medium Derived from Nanog-Introduced Amniotic Fluid-Derived MSCs

A cell portion of each of the amniotic fluid-derived MSC lines established in Examples 1 and 2 was selected, 100 ml of a conditioned medium thereof was produced, and then exosomes were isolated therefrom.

The extraction of exosomes from the conditioned medium was performed using an Amicon Ultra-15 10K centrifugal filter device (Millipore), which was devised to filter certain molecular-weight proteins of the conditioned medium produced from the fetus-derived MSCs. 14.8 ml of the conditioned medium was placed in an upper cap of the device, and then centrifuged at 5,000 x g for 15 minutes to 40 minutes. 100 ml of the conditioned medium was concentrated in this manner, and then an exosome isolation kit (Invitrogen) was added to the concentrated medium in an amount of 1/2 of the final volume and then mixed well. The completed samples were stored at 4 °C for 12 hours. Thereafter, each sample was centrifuged at 13,200 rpm for 30 minutes to remove the supernatant, and then the remaining white pellet was dissolved with PBS or DMEM, thereby completing the preparation of exosome samples.

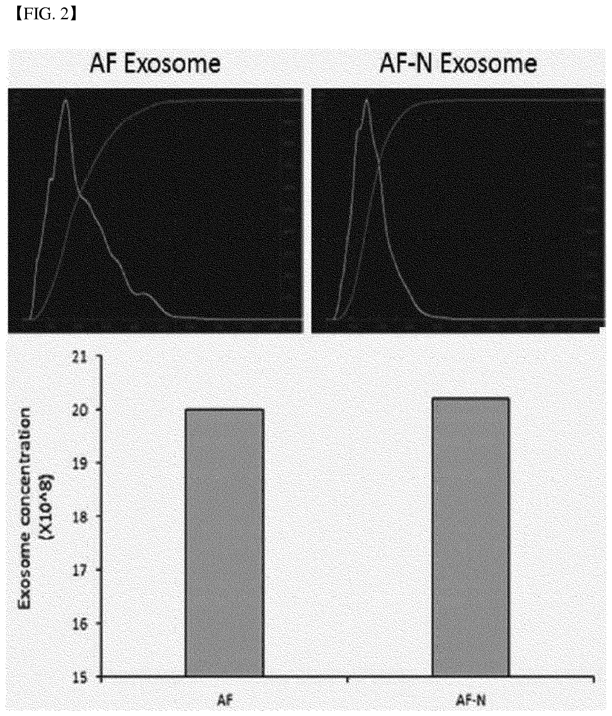

As a result of measuring the average size and number of exosomes using Nanosight, the average sizes of amniotic fluid-derived MSCs and the Nanog-introduced amniotic fluid-derived MSCs were recorded as diameters of 156 nm and 147 nm, respectively, and the numbers thereof were measured as 20.0 x 10⁸ and 20.1 x 10⁸, respectively (see FIG. 2). That is, it was confirmed that the isolation of exosomes from the stem cell lines used in the experiments was normally carried out, and it was also possible to isolate the same amount of exosomes from the Nanog-introduced amniotic fluid-derived MSCs.

### Example 4: Analysis of Substances in Exosomes

To analyze internal substances of the exosomes isolated in Example 3, first, the content of mRNA was examined by RT-PCR.

For qRT-PCR, RNA was obtained in the same manner as the RNA prep during RT-PCR, and cDNA was prepared in the same manner. The prepared cDNA was subjected to qRT-PCR through a CFX-96 PCR system, and was amplified using primers specific to mRNA of bFGF, IGF, Wnt7a, and PDGF-AA. The bFGF-specific primers used were bFGF forward: 5'-CAGATTAGCGGACGCGGTGC-3' (SEQ ID NO: 4) and bFGF reverse: 5'-TCACGGATGGGTGTCTCCGC-3' (SEQ ID NO: 5), the IGF-specific primers used were IGF forward: 5'-CCATGTCCTCCTCGCATCTCTTCT-3' (SEQ ID NO: 6) and IGF reverse: 5'-CCATACCCTGTGGGCTTGTTGAA-3' (SEQ ID NO: 7), the Wnt7a-specific primers used were Wnt7a forward: 5'-TCTTTCTCAGCCTGGGCATGGT-3' (SEQ ID NO: 8) and Wnt7a reverse: 5'-TCCTATGACGATGATGGCGTCG-3' (SEQ ID NO: 9), and the PDFG-AA-specific primers used were PDGF-AA forward: 5'-CTGCCCATTCGGAGGAAGAGAA-3' (SEQ ID NO: 10) and PDGF-AA reverse: 5'-TGGCACTTGACACTGCTCGTGTT-3' (SEQ ID NO: 11). Relative quantification of target mRNA was analyzed by a CT method.

To examine the secretion of bFGF, Wnt7a and PDGF-AA proteins, an enzyme-linked immunosorbent assay (ELISA assay) was performed on exosomes isolated from the Nanog-introduced amniotic fluid-derived MSCs. The contents of bFGF, Wnt7a, and PDGF-AA proteins in the respective exosomes were determined using an ELISA kit (RayBiotech). For the ELISA, exosomes were prepared, a standard and a sample were treated with biotin antibodies for 1 hour and then treated with streptavidin for 45 minutes. Subsequently, the standard and sample were treated with a TMB substrate reagent for 30 minutes, and then a stop solution was added thereto to stop the reaction. The results were quantitatively analyzed by measuring absorbance at 450 nm using a microplate spectrophotometer.

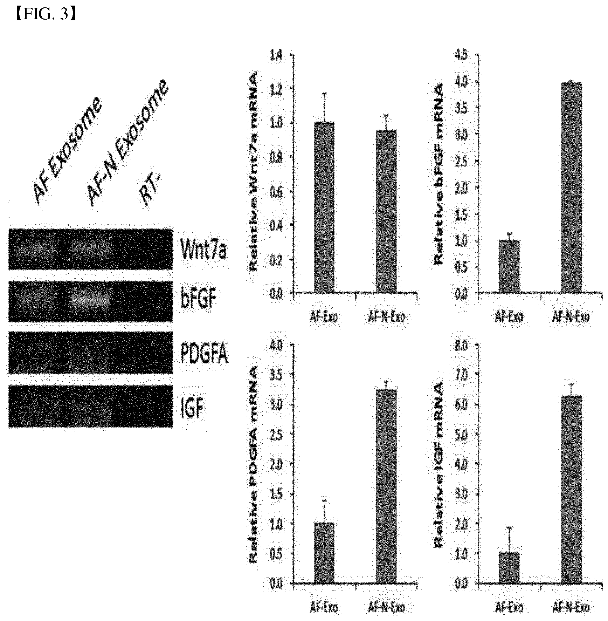

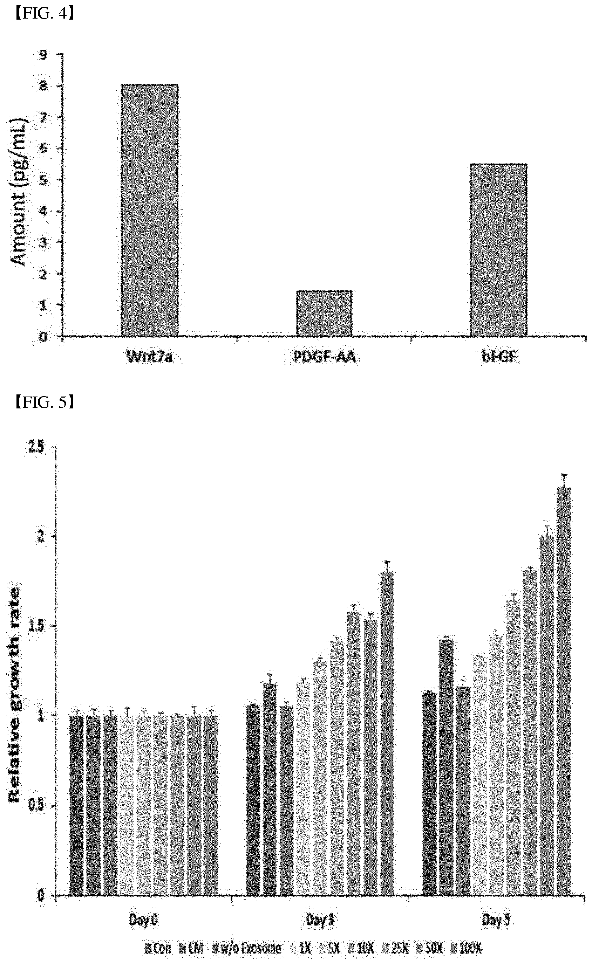

As a result of comparing the expression levels of hair growth-related genes through RT-PCR, the Nanog-introduced amniotic fluid-derived MSC exosomes exhibited higher expression levels for bFGF, PDGF, and IGF except for Wnt7a. This indicates that the number of exosomes from the Nanog-introduced amniotic fluid-derived MSCs was similar to that of existing stem cells, but there was a slight difference in internal substances of the exosomes between the two cases. To more accurately examine RT-PCR data, quantitative analysis was performed through qRT-PCR. Similar to the case of RT-PCR, it was confirmed that mRNA levels of the other growth factors except for Wnt7a were considerable (see FIG. 3). In conclusion, mRNA of hair growth-related factors is included in exosomes, which may be interpreted as aiding in promoting hair growth. The amounts of the proteins were analyzed by ELISA, and the concentrations of Wnt7a, PDGF-AA, and bFGF were measured (see FIG. 4). According to measurement results, the contents of Wnt7a, PDGF-AA, and bFGF were about 8 pg/mL, 2.5 pg/mL, and about 5.5 pg/mL, respectively. From the results, it was confirmed that the hair growth-related proteins were also included in the exosomes.

### Example 5: Growth and Marker Changes of Dermal Papilla Cells through Exosome Treatment

After the obtained exosomes were divided into various concentrations, dermal papilla cells were treated therewith to confirm two factors that play a crucial role in hair generation, i.e., the growth of dermal papilla cells and changes in hair growth markers. Concentrations used ranged from 100X to 1X and comparative analysis was carried out using a conditioned medium and an exosome-free conditioned medium.

Specifically, skin tissues of mice were collected and cells were isolated therefrom, total RNA was isolated from the cells using the same method as that used in Example 2 and reverse-transcribed with cDNA, and cDNA was amplified using a Taq polymerase (Promega) and primers specific to mRNA of ALP, LEF, and Versican. The ALP-specific primers used were ALP forward: 5'-TGGCCCTCTCCAAGACGTACAA-3' (SEQ ID NO: 12) and ALP reverse: 5'-TGGTTCACTCTCGTGGTGGTCA-3' (SEQ ID NO: 13), the LEF-specific primers used were LEF forward: 5'-CTTCCTTGGTGAACGAGTCTG-3' (SEQ ID NO: 14) and LEF reverse: 5'-GTGTTCTCTGGCCTTGTCGT-3' (SEQ ID NO: 15), and the Versican-specific primers used were Versican forward: 5'-AACTAGCCGTTGGAGTGGATTC-3' (SEQ ID NO: 16) and Versican reverse: 5'-AAATGCTCTGTGGCTCTGGA-3' (SEQ ID NO: 17). The resulting product was analyzed by electrophoresis on 1% agarose gel, and a relative mRNA expression level was quantified based on GAPDH mRNA by CT value-based analysis.

According to the results, the growth of dermal papilla cells was gradually increased as the concentration was increased, and a much better growth promotion ability was exhibited at a certain concentration or higher, compared to that in a conditioned medium. In addition, it was confirmed that the exosome-removed conditioned medium exhibited significantly reduced functionality, which suggests that the functions of exosomes account for a large part also in the conditioned medium (see FIG. 5).

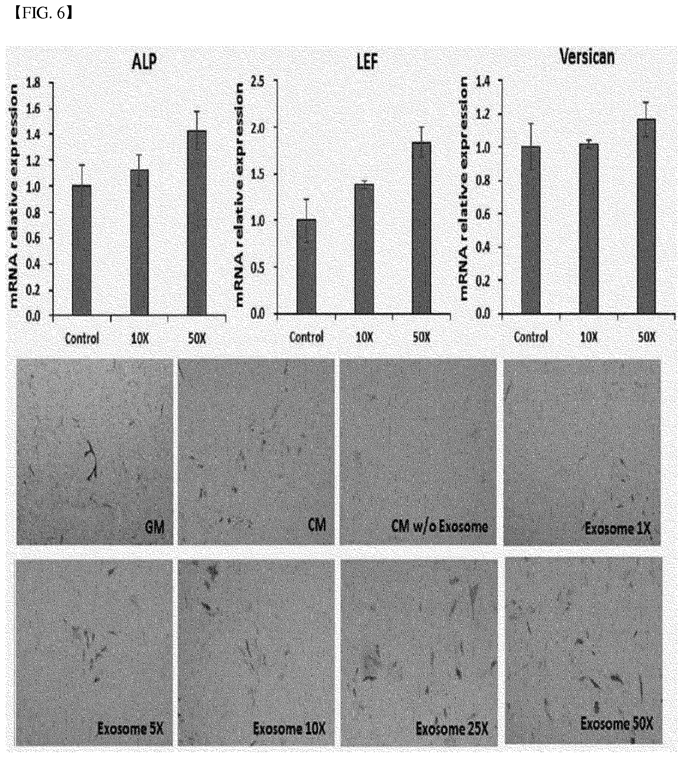

In addition to the growth of dermal papillae, changes in hair growth markers were confirmed through qRT-PCR and AP staining. In order for the dermal papilla cells to induce hair growth, it is necessary to maintain intrinsic sternness of dermal papilla cells, and ALP, LEF, and Versican are typically known as markers representing this sternness. Thus, as a result of distinguishing the expression levels of these markers in an exosome-free medium (Control) and various concentrations (10X and 50X), the expression levels of ALP and LEF were confirmed to be increased 1.5-fold to 2-fold (see upper side of FIG. 6). In the case of Versican, it seemed to be a small increase but not much different. To obtain reliability of this data, AP staining for confirming ALP by staining was carried out, and it was confirmed that, similar to the case of qRT-PCR, the number of cells stained blue was increased as the concentration of exosomes was increased (see lower side of FIG. 6).

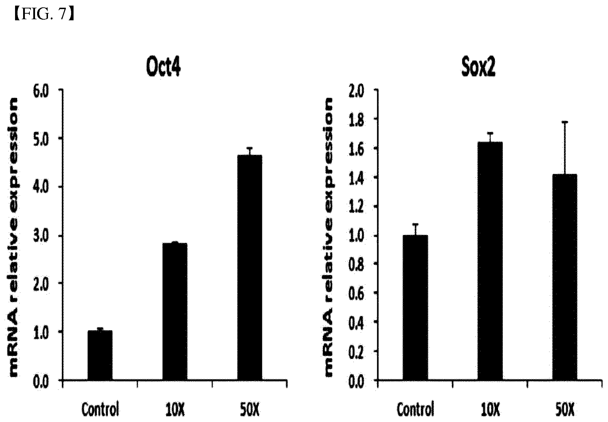

Additionally, since dermal papilla cells are also known to have some degree of functionality as mesenchymal stem cells, they express the common stem cell markers Oct4 and Sox2. As a result of confirming changes in general stem cell markers, a gradual increase was exhibited, as with ALP and LEF (see FIG. 7). In particular, Oct4 exhibited a 5-fold or higher difference, which may be interpreted that exosomes have a great influence on Oct4 expression.

From these results, it may be determined that exosomes have an efficient effect on hair growth by promoting a hair growth ability of dermal papilla cells.

### <Preparation Example 1> Preparation of Hair Lotion

According to a conventional hair lotion preparation method, a hair lotion including, as an active ingredient, exosomes in a conditioned medium of Nanog-introduced amniotic fluid-derived MSCs of the present invention was prepared as shown in Table 1.

**[Table 1]**

| **Ingredients to be mixed** | **Content (wt%)** |
|---|---|
| Exosomes isolated from Nanog-introduced amniotic fluid-derived MSCs | 5.0 |
| resorcinol | 2.0 |
| Panthenol | 0.5 |
| Pyrrothonol amine | 0.1 |
| Pigment | Appropriate amount |
| Fragrance | Appropriate amount |
| Purified water | Balance |
| Total | 100 |

### <Preparation Example 2> Preparation of Hydrophilic Ointment

According to a conventional hydrophilic ointment preparation method, a hydrophilic ointment including, as an active ingredient, exosomes in a conditioned medium of Nanog-introduced amniotic fluid-derived MSCs of the present invention was prepared as shown in Table 2.

**[Table 2]**

| **Ingredients to be mixed** | **Content (wt%)** |
|---|---|
| Exosomes isolated from Nanog-introduced amniotic fluid-derived MSCs | 0.5 |
| Ethyl (or methyl) p-oxybenzoate | 0.25 |
| Lauryl sodium sulfate | 15 |
| Propyl p-oxybenzoate | 0.15 |
| White vaseline | Appropriate amount |
| Stearyl alcohol | Appropriate amount |
| Propylene glycol | Balance |
| Total | 100 |

## Claims

1. A method of producing a mesenchymal stem cell-derived exosome, the method comprising:
(a) isolating a fetus-derived cell from amniotic fluid obtained from a pregnant woman;
(b) sub-culturing the isolated fetus-derived cell in a medium containing fetal bovine serum (FBS) and basic fibroblast growth factor (bFGF) to obtain a mesenchymal stem cell derived from a fetus in amniotic fluid;
(c) introducing Nanog into the mesenchymal stem cell derived from a fetus in amniotic fluid to obtain an Nanog-overexpressing mesenchymal stem cell derived from a fetus in amniotic fluid;
(d) culturing the obtained Nanog-overexpressing mesenchymal stem cell derived from a fetus in amniotic fluid for 1 day to 5 days to prepare a conditioned medium; and
(e) isolating an exosome from the conditioned medium,
wherein the isolated exosome comprises any one or more human growth factors selected from the group consisting of basic fibroblast growth factor (bFGF), insulin-like growth factor (IGF), Wingless-type MMTV integration site family member 7A (Wnt7a), and platelet-derived growth factor (PDGF)-AA.

2. The method of claim 1, wherein, in process (c), the Nanog is introduced via a retroviral vector.

3. The method of claim 1, wherein the sub-culturing is performed in a medium further comprising selenium and ascorbic acid.

4. A cosmetic composition for promoting hair growth, the cosmetic composition prepared using the method of any one of claims 1 to 3 and comprising a mesenchymal stem cell-derived exosome as an active ingredient.

5. A pharmaceutical composition for promoting hair growth, the pharmaceutical composition prepared using the method of any one of claims 1 to 3 and comprising a mesenchymal stem cell-derived exosome as an active ingredient.
